# EUROPEAN PATENT SPECIFICATION

(11) **EP 1 809 383 B1**
(45) Date of publication and mention of the grant of the patent: **30.07.2008**
(21) Application number: 05792911.9
(22) Date of filing: 13.10.2005
(51) Int. Cl.: A61Q 19/00, A61Q 19/08, A61Q 19/10, A61Q 17/04, A61K 8/44, A61K 8/67, A61K 8/97

(54) **COSMETIC COMPOSITIONS**
KOSMETISCHE ZUSAMMENSETZUNGEN
FORMULES COSMÉTIQUES

(30) Priority: 19.10.2004 GB 0423164; 31.08.2005 US 712396 P
(43) Date of publication of application: 25.07.2007
(73) Proprietor: The Boots Company PLC, Nottingham, Nottinghamshire NG2 3AA (GB)
(72) Inventor: BARTON, Stephen Peter, Chilwell,Nottingham, NG9 4DF (GB); LONG, Stewart Paul, Oakham,Rutland LE15 6NF (GB); HAMILTON, Lloyd George, Stapleford,Nottingham NG9 7GL (GB)
(74) Representative: Jones, Stephen Anthony
(86) International application number: PCT/GB2005/003946
(87) International publication number: WO 2006/043033

(56) References cited:
- FR-A- 2 746 008
- FR-A- 2 768 335
- US-A- 5 690 918
- US-A1- 2002 044 913
- US-A1- 2004 176 448

## Description

This invention relates to cosmetic compositions, and in particular to cosmetic compositions for application to the skin.

Improvements in energy production and utilisation in the skin may lead to numerous benefits. These may result from:
- the way the skin feels (freshened, invigorated or toned sensations);
- the way the skin looks (increased radiance, reduced dullness or firmer appearance);
- the way the skin behaves (less reactive to potentially harmful stimuli, reduced occurrence of problem skin or improvements in elasticity).

These benefits may be particularly important in older skin, especially on exposed areas, as such skin tends to have a dry, dull, uneven complexion and is less effective at dealing with minor trauma.

The underlying physiology of energy production and utilisation in the skin is complex. In simple terms, it can be seen as having two phases:
1. provision of nutrients to the skin via the blood flow; and
2. conversion of these nutrients into new tissue and sustained tissue function.

The first phase is "energising" in that increases in blood and nutrient better enable the skin to maintain its functions. Again, this may be particularly true of older skin, for which blood vessels in the skin can be reduced in number and effectiveness, especially where chronic sun damage has occurred. Improvements in blood flow may also result in a feeling of invigoration.

The second phase is more complex. Typically, improvement in the production of adenosine triphosphate (ATP) may be used as an *in vitro* assay of an "energising" effect of a cosmetic. ATP is the molecule that the body uses to store energy. However, utilisation of energy is as important as generation of energy, and this involves passage of electrons along a chain of molecules. In this process, molecules such as Nicotine Adenine Dinucleotide (NAD/NADH) reversibly transfer hydrogen ions. Resazurin, a non-toxic dye, may be used to monitor this process, as it is converted to a red colour when it takes up hydrogen ions lost in the conversion of NADH to NAD. Changes in the rate of resazurin reduction by cells may therefore be used to visualise the rate of energy utilisation.

Old skin and young skin show similar rates of energy production and utilisation when at rest. However, when the skin is subjected to stress (as may occur, for instance, as a result of exposure to UV radiation, dehydration, or numerous other causes), utilisation of energy is reduced in older skin. Improving the responsiveness of skin, particularly older skin, to stress may therefore lead to the benefits referred to above, particularly in terms of improved appearance of the skin.

There have now been devised cosmetic compositions comprising combinations of active ingredients, that lead to some or all of the benefits associated with energising of the skin that are referred to above, and which may be attributable to improvements in the generation and/or utilisation of energy in skin that has been subjected to stress.

Thus, according to the invention, there is provided a cosmetic composition comprising two or more active ingredients selected from the group consisting of
a) dipalmitoyl hydroxyproline, salts and esters thereof;
b) carnitine; and
c) an extract of *Haematococcus pluvialis.*

The composition according to the invention is advantageous primarily in that its use leads to cosmetic skincare benefits associated with energising of the skin. Such benefits may be perceived by a user as a freshened, invigorated or toned feel of the skin, reduced dullness or firmer appearance of the skin, and/or a greater tolerance of the skin to potentially harmful stimuli. These benefits may be particularly pronounced when the composition is applied to the skin of older users. The composition may be particularly useful in protecting the skin from the adverse effects of external stress-inducing stimuli such as exposure to UV radiation, factors inducing dehydration of the skin.

One preferred group of compositions according to the invention comprise dipalmitoyl hydroxyproline in combination with carnitine.

Another preferred group of compositions according to the invention comprise dipalmitoyl hydroxyproline in combination with an extract of *Haematococcus pluvialis.*

The compositions according to the invention preferably further comprise one or more vitamins. Such vitamins preferably include one or more of
a) ascorbic acid (vitamin C), its salts, esters, glucosides and glucosamines, particularly sodium ascorbyl phosphate, magnesium ascorbyl phosphate and ascorbyl palmitate; and
b) tocopherol (vitamin E) and its esters, particularly tocopheryl acetate.

Where dipalmitoyl hydroxyproline is present in the composition, it is preferably present in an amount from 0.1% to 10% w/w, more preferably from 0.25% to 5% w/w.

Where carnitine is present in the composition, it is preferably present in an amount from 0.025% to 0.75% w/w, more preferably from 0.05% to 0.5% w/w.

Where an extract of *Haematococcus pluvialis* is present in the composition, it is preferably present in an amount of from 0.005% to 0.1 % w/w, more preferably from 0.01% to 0.07% w/w.

Where vitamin C is present in the composition, it is preferably present in an amount of from 0.1 to 5% w/w, more preferably from 0.2% to 2% w/w.

Where vitamin E is present in the composition, it is preferably present in an amount of from 0.01 % and 1% w/w, more preferably from 0.05% to 0.5% w/w.

Dipalmitoyl hydroxyproline is most preferably used in the form of the product sold under the trade name SEPILIFT DPHP by S.E.P.P.I.C., 75 Quai d'Orsay, 75321 Paris cedex 07, France.

Carnitine is most preferably used in the form of the product sold under the trade name COAXEL by Sederma Inc of 7 Century Drive, Parsippany, NJ 07054, USA. The COAXEL product comprises carnitine (5% w/w) in admixture with *inter alia* caffeine (1% w/w) and coenzyme A (0.015% w/w).

The extract of *Haematococcus pluvialis* is most preferably used in the form of the product sold under the trade name HYDRERGY by Sederma Inc of 7 Century Drive, Parsippany, NJ 07054, USA. The HYDRERGY product comprises *Haemococcus pluvialis* extract (1% w/w) in admixture with *inter alia* polyglucuronic acid (0.15% w/w).

Suitable cosmetic compositions include colour cosmetics such as lipsticks, foundations, lip balms, face creams, toner cleansers, aftersuns, moisturisers, and face masks. Suitable formulation types include gels, creams, serums, pastes, lotions, milks, ointments, salves, sticks, spray, roll-on, powders, solutions, suspension dispersions and emulsions, be they w/o, o/w, w/o/w or o/w/o.

One group of preferred cosmetic compositions according to the invention include one or more sunscreen agents.

The sunscreen agents may comprise organic or inorganic sun filters or a combination of the two. Suitable inorganic sunfilters include:
a) Microfine titanium dioxide
b) Microfine zinc oxide
c) Boron nitride

Suitable organic sunscreens include:
a) p-aminobenzoic acids, their esters and derivatives (for example, 2-ethylhexyl p-dimethylaminobenzoate),
b) methoxycinnamate esters (for example, 2-ethylhexyl p-methoxycinnamate, 2-ethoxyethyl p-methoxycinnamate or α,β-di-(p-methoxycinnamoyl)-α'-(2-ethylhexanoyl)-glycerin,
c) benzophenones (for example oxybenzone),
d) dibenzoylmethanes such as 4-(tert-butyl)-4'-methoxydibenzoylmethane,
e) 2-phenylbenzimidazole-5 sulfonic acid and its salts,
f) alkyl-β,β-diphenylacrylates, for example alkyl α-cyano-β,β-diphenylacrylate such as octocrylene,
g) triazines such as 2,4,6-trianilino-(p-carbo-2-ethylhexyl-1-oxo)-1,3,5 triazine,
h) camphor derivatives such as methylbenzylidene camphor

Any sunscreening agent is preferably present in an amount from 0.1 to 10% by weight of the composition.

Sunscreen compositions may be formulated as any suitable form, as known to those skilled in the art. Particularly preferred formulation types are emulsions and oily dispersions.

Further components may be added to the composition as is well-known to those skilled in the art.

Suitable oils for the oil phase of the oily dispersions and the oil phase of the water-in-oil and oil-in-water emulsions of the present invention may comprise for example:
a) hydrocarbon oils such as paraffin or mineral oils;
b) waxes such as beeswax or paraffin wax;
c) natural oils such as sunflower oil, apricot kernel oil, shea butter or jojoba oil;
d) silicone oils such as dimethicone, cyclomethicone or cetyldimethicone;
e) fatty acid esters such as isopropyl palmitate or isopropyl myristate;
f) fatty alcohols such as cetyl alcohol or stearyl alcohol; or
g) mixtures thereof, for example, the blend of waxes available commercially under the trade name Cutina (Henkel).

The emulsifiers used may be any emulsifiers known in the art for use in water-in-oil or oil-in-water emulsions. It has been found that particularly effective water-in-oil and oil-in-water sunscreen compositions can be prepared by using an emulsifier or mixture of emulsifiers selected from known cosmetically acceptable emulsifiers which include:
a) sesquioleates such as sorbitan sesquioleate, available commercially for example under the trade name Arlacel 83 (ICI), or polyglyceryl-2-sesquioleate;
b) ethoxylated esters of derivatives of natural oils such as the polyethoxylated ester of hydrogenated castor oil available commercially for example under the trade name Arlacel 989 (ICI);
c) silicone emulsifiers such as silicone polyols available commercially for example under the trade name ABIL WS08 (Th. Goldschmidt AG);
d) anionic emulsifiers such as fatty acid soaps e.g. potassium stearate and fatty acid sulphates e.g. sodium cetostearyl sulphate available commercially under the trade name Dehydag (Henkel);
e) ethoxylated fatty alcohols, for example the emulsifiers available commercially under the trade name Brij (ICI);
f) sorbitan esters, for example the emulsifiers available commercially under the trade name Span (ICI);
g) ethoxylated sorbitan esters, for example the emulsifiers available commercially under the trade name Tween (ICI);
h) ethoxylated fatty acid esters such as ethoxylated stearates, for example the emulsifiers available commercially under the trade name Myrj (ICI);
i) ethoxylated mono-, di-, and tri-glycerides, for example the emulsifiers available commercially under the trade name Labrafil (Alfa Chem.);
j) non-ionic self-emulsifying waxes, for example the wax available commercially under the trade name Polawax(Croda);
k) ethoxylated fatty acids, for example, the emulsifiers available commercially under the trade name Tefose (Alfa Chem.); or
l) mixtures thereof.

Additional skincare actives known to have benefit on the skin may also be added. Examples include anti-acne actives eg salicylic acid, vitamins eg vitamin A, vitamin E, vitamin C, and their derivatives, peptides such as di-, tri-, tetra-, penta- and hexapeptides that are known to have a variety of effects on skin such as wrinkle reduction and collagen boosting, hydroxy acids, anti-oxidants, anti-inflammatories, anti-microbials eg triclosan, skin-lightening actives, anti-fungals, skin conditioners eg panthenol, and other suitable materials that are known to those skilled in the art.

Preservatives may be added to the composition, such as 2-bromo-2-nitropropane-1,3-diol (bronopol, which is available commercially under the trade name Myacide RTM), benzyl alcohol, diazolidinyl urea, imidazolidinyl urea, methyl paraben, ethyl paraben, phenoxyethanol, propyl paraben, sodium methyl paraben, sodium ethyl paraben, sodium dehydroacetate, polyhexamethylenebiguanide hydrochloride, methyl isothiazolinone, methyl chloroisothiazolinone, DMDM hydantoin, iodopropynyl butylcarbamate and sodium propyl paraben, suitably in an amount of from 0.01% to 10% by weight of the composition.

Thickeners, viscosity modifying agents and/or gelling agents may be added to the composition, such as acrylic acid polymers eg available commercially under the trade name Carbopol (B.F. Goodrich) or modified celluloses eg hydroxyethylcellulose available commercially under the trade name Natrosol (Hercules) or hydroxypropylmethyl cellulose, amine oxides, block polymers of ethylene oxide and propylene oxide (for example, those available from BASF Wyandotte under the trade name "Pluronic" RTM), PVM, MA, or a decadiene crosspolymer (available under the trade name Stabilez 60), ethoxylated fatty alcohols, salt (NaCl), phthalic acid amide, polyvinyl alcohols, fatty alcohols and alkyl galactomannans available under the trade name N-Hance from Hercules, suitably in an amount of from 0.5% to 10% by weight of the composition.

Sequestering agents may be added to the composition, such as ethylenediamine tetraacetic acid and salts thereof, suitably in an amount of from 0.005% to 0.5% by weight of the composition.

The composition may also include waxes such as cocoa butter, suitably in an amount of from 1% to 99% by weight of the composition.

The composition may also comprise suitable cosmetically acceptable diluents, carriers and/or propellants such as dimethyl ether.

Perfumes may be added suitably in an amount of from 0.01 % to 2% by weight of the composition, as may water soluble dyes such as tartrazine, suitably in an amount of from a trace amount (such as 1 x 10⁻⁵ %) to 0.1 % by weight of the composition.

The composition may also include pH adjusting agents such as sodium hydroxide, aminomethyl propanol, triethanolamine, suitably in an amount of from 0.01% to 10% by weight of the composition.

The composition may be buffered by means well known in the art, for example by use of buffer systems comprising succinic acid, citric acid, lactic acid, and acceptable salts thereof, phosphoric acid, mono- or disodium phosphate and sodium carbonate. Suitably, the composition may have a pH between 3 and 10, preferably between 4 and 8. Surfactants may be included, such as cosmetically acceptable salts of alkyl ether sulphates, alkyl and alkylamidoalkyl betaines, ethoxylated alcohols, polyethylene glycol carboxylates, aceceptable salts of alkyl sulphates (such as ammonium lauryl sulphate), acceptable salts of alkyl ether sulphates (such as ammonium laureth sulphate or sodium laureth sulphate), sulphosuccinates (such as disodium laureth sulphosuccinate), amphoacetates and amphodiacetates (such as cocoamphodiacetate), alkylpolyglucosides and alcohol sulphonates.

The compositions of the present invention may additionally comprise other components which will be well known to those skilled in the art. These include, for example, emollients such as isopropyl myristate or triglycerides of fatty acids eg lauric triglyceride or capric/caprylic triglyceride, such as the triglyceride available commercially under the trade name Miglyol 810 (Huls UK); moisturisers such as D-panthenol; humectants such as glycerin or 1,3-butylene glycol; antioxidants such as DL-α-tocopheryl acetate or butylated hydroxytoluene; emulsion stabilising salts such as sodium chloride, sodium citrate or magnesium sulphate; film formers to assist spreading on the surface of the skin such as alkylated polyvinylpyrrolidone eg available commercially under the trade name Antaron (GAF) and colourings.

The invention will now be described in greater detail, by way of illustration only, with reference to the following Examples, in which the ingredients referred to by the trade names SEPILIFT DPHP, HYDRERGY and COAXEL are as described above.

### Example 1 - Anti-Ageing Skin Cream

| | %w/w |
|---|---|
| Aqua | to 100 |
| Potassium hydroxide | 0.03 |
| Tetrasodium EDTA | 0.05 |
| Ethylhexyl methoxycinnamate | 7.5 |
| Glycerin | 7 |
| C12-C15 alkyl benzoate | 5 |
| Dimethicone | 2.5 |
| Butyl methoxydibenzoylmethane | 3 |
| Butyrospermum parkii | 3 |
| Ethylhexyl salicylate | 2 |
| Polyglyceryl-3 methylglucose distearate | 2 |
| Polyacrylamide emulsion | 1.50 |
| Cetyl alcohol | 1 |
| PVP/hexadecene copolymer | 1 |
| Acrylates/Vinyl Isodecanoate Copolymer | 0.1 |
| Cyclopentasiloxane | 0.5 |
| Dimethiconol | 0.5 |
| C18-36 acid glycol ester | 0.5 |
| Butylene Glycol | 0.3 |
| Palmitoyl oligopeptide | 0.00015 |
| Palmitoyl tetrapeptide-3 | 0.00015 |
| Hydrergy | 3.00 |
| Sepilift | 0.5 |
| Sodium ascorbyl phosphate | 0.25 |
| Preservative | q.s |
| Perfume | q.s |

### Method

### Stage 1

Mix together ethylhexyl methoxycinnamate, C12-C15 alkyl benzoate, dimethicone, butyl methoxydibenzoylmethane, Butyrospermum parkii, ethylhexyl salicylate, polyglyceryl-3 methylglucose distearate, cetyl alcohol, PVP/hexadecene copolymer, C18-36 acid glycol ester, Sepilift and heat to 70-75°C to melt the waxes. Add acrylates/vinyl isodecanoate copolymer and homogenise for 2 minutes.

### Stage 2

Add glycerin, potassium hydroxide, tetrasodium EDTA and butylene glycol to water and heat to 70-75°C.

### Stage 3

Add Stage 1 to stage 2 and homogenise for 2 minutes. Add Cyclopentasiloxane and dimethiconol and stir. Cool to below 40°C.

### Stage 4

Add palmitoyl oligopeptide, palmitoyl tetrapeptide-3, Hydrergy, sodium acsorbyl phosphate, preservative and perfume and mix. Add polyacrylamide and mix until uniform.

### Example 2 - Aftersun Lotion

| | %w/w |
|---|---|
| Aqua | to 100 |
| Hydrated silica | 5 |
| Isopropyl palmitate | 4 |
| Arachidyl propionate | 2 |
| Dimethicone | 2 |
| Glycerin | 2 |
| Steareth-21 | 1.96 |
| Steareth-2 | 1.683 |
| Cetyl alcohol | 1 |
| Tribehenin | 1 |
| Glyceryl stearate | 1 |
| Paraffinum liquidum | 0.994 |
| Panthenol | 0.75 |
| Parfum | q.s |
| Xanthan gum | 0.3 |
| Sodium citrate | 0.25 |
| Tocopheryl acetate | 0.2 |
| Hydroxyethylcellulose | 0.1 |
| Bisabolol | 0.095 |
| Citric acid | 0.05 |
| Preservative | q.s |
| Hydrergy | 5 |
| Sepilift DPHP | 3 |
| Sodium Ascorbyl Phosphate | 1 |

### Method

### Stage 1

The citric acid, sodium citrate and hydroxyethylcellulose are added to the water. Using a propellor stirrer, the mixture is stirred until dispersed. The xanthan gum is pre-dispersed in the glycerin and this is then added to the bulk, which is then heated to 70°C.

### Stage 2

The isopropyl palmitate, arachidyl propionate, dimethicone, steareth-21, steareth-2, cetyl alcohol, tribehenin, sepilift DPHP, glyceryl stearate, paraffinum liquidum are mixed and heated to 70°C to melt the waxes.

### Stage 3

Using a homogeniser, stage 2 is added to stage 1 and is mixed until emulsified and uniform. The emulsion is cooled to below 35° C using stirring. Once below 35°C, the remaining materials are added, including the Hydrergy and Sodium Ascorbyl Phosphate, and mixed until uniform.

### Example 3 - Day Cream

| | %w/w |
|---|---|
| Aqua | to 100 |
| Butylene glycol | 5 |
| Dicaprylyl maleate | 4 |
| Paraffinum liquidum | 4 |
| Octyl methoxycinnamate | 3 |
| Petrolatum | 3 |
| Cetyl Alcohol | 2 |
| Glycerin | 2 |
| Dimethicone | 2 |
| Cetearyl alcohol | 1.6 |
| Butyl methoxydibenzoylmethane | 1 |
| Hydroxyethylcellulose | 0.4 |
| PEG-20 stearate | 0.4 |
| Polyacrylamide (Sepigel 305 ex Seppic) | 1.50 |
| Parfum | q.s |
| Retinyl palmitate | 0.15 |
| Tetrasodium EDTA | 0.1 |
| Citric acid | 0.08 |
| BHT | 0.0024 |
| Hydrergy | 3 |
| Sepilift DPHP | 0.5 |
| Sodium Ascorbyl Phosphate | 0.25 |
| Preservative | q.s |

### Method

### Stage 1

Tetrasodium EDTA and citric acid are added to the water using a propellor stirrer. The hydroxyethylcellulose is added and dispersed using a homogeniser. Butylene glycol, glycerin and methylparaben are added and the bulk is heated to 70°C.

### Stage 2

The dicaprylyl maleate, paraffinum Liquidum, octyl methoxycinnamate, petrolatum, cetyl alcohol, dimethicone, cetearyl alcohol, butyl methoxydibenzoylmethane, PEG-20 stearate, Sepilift DPHP, and BHT are mixed and heated to 70°C to melt the waxes.

### Stage 3

Using a homogeniser, stage 2 is added to stage 1 and the bulk is mixed until emulsified and stable. The product is then cooled to below 35°C using stirring. The remaining raw materials, including the Hydrergy and Sodium Ascorbyl Phosphate are added and the product is mixed using a propellor stirrer until uniform. Add Polyacrylamide and mix until uniform.

### Example 4 - Sun Lotion SPF8

| | %w/w |
|---|---|
| Aqua | to 100 |
| C12-15 Alkyl Benzoate | 8 |
| Butylene glycol | 5 |
| Butyl methoxydibenzoylmethane | 2.2 |
| Dimethicone | 2 |
| Polyglyceryl-3 methylglucose distearate | 2 |
| PVP/hexadecene copolymer | 1.75 |
| Octyl methoxycinnamate | 1.7 |
| Theobroma cacao | 0.5 |
| Parfum | q.s |
| Tocopheryl acetate | 0.2 |
| Acrylates/vinyl isodecanoate crosspolymer | 0.15 |
| Potassium hydroxide | 0.034 |
| Tetrasodium EDTA | 0.02 |
| Preservative | q.s |
| Hydrergy | 3 |
| Sepilift DPHP | 1.5 |
| Sodium Ascorbyl Phosphate | 0.75 |

### Method

### Stage 1

The EDTA is dispersed into the water. Using a propellor stirrer, the acrylates/vinyl isodecanoate crosspolymer are added and dispersed and hydrated. Butylene glycol is added and the aqueous phase is heated to 70°C.

### Stage 2

The C12-15 alkyl benzoate, butyl methoxydibenzoylmethane, dimethicone, polyglyceryl-3 methylglucose distearate, Sepilift DPHP, PVP/hexadecene copolymer, octyl methoxycinnamate, theobroma cacao and tocopheryl acetate are mixed and heated to 70°C to melt the waxes.

### Stage 3

Using a homogeniser, stage 2 is added to stage 1 and the bulk is mixed until emulsified and uniform. The emulsion is cooled to below 35°C with stirring. The remaining materials, including the Hydrergy, Sodium Ascorbyl Phosphate are added and mixed until uniform.

### Example 5 - Aftersun Treatment

| | %w/w |
|---|---|
| Aqua | to 100 |
| Petrolatum | 3 |
| Cetyl Alcohol | 2 |
| Dimethicone | 2 |
| Glycerin | 2 |
| Ceteath-20 | 1.7 |
| Paraffinum Liquidum | 1 |
| Sodium chloride | 0.8 |
| Theobroma cacao | 0.7 |
| Glyceryl stearate | 0.5 |
| Parfum | q.s |
| Allantoin | 0.2 |
| Hydroxyethylcellulose | 0.1 I |
| Triclosan | 0.1 |
| Citric acid | 0.02 |
| Preservative | q.s |
| CoAxel | 5 |
| Sepilift DPHP | 3.5 |
| Sodium Ascorbyl Phosphate | 1.25 |

### Method

### Stage 1

Into the water, sodium chloride and citric acid are added and dispersed. Using a propellor stirrer, hydroxyethylcellulose is added and dispersed. This phase is then heated to 70°C.

### Stage 2

The petrolatum, cetyl alcohol, Sepilift DPHP, dimethicone, ceteath-20, paraffinum liquidum, theobroma cacao and glyceryl stearate are mixed and heated to 70°C to melt the waxes.

### Stage 3

Using a homogeniser, stage 2 is added to stage 1, this is mixed until emulsified and uniform. The emulsion is then cooled to below 35°C with stirring. The remaining materials, including the CoAxel, Sodium Ascorbyl Phosphate are then added and mixed until uniform.

### Example 6 - Aftersun Treatment

| | %w/w |
|---|---|
| Aqua | to 100 |
| Petrolatum | 3 |
| Cetyl Alcohol | 2 |
| Dimethicone | 2 |
| Glycerin | 2 |
| Ceteath-20 | 1.7 |
| Paraffinum Liquidum | 1 |
| Sodium chloride | 0.8 |
| Theobroma cacao | 0.7 |
| Glyceryl stearate | 0.5 |
| Parfum | q.s |
| Allantoin | 0.2 |
| Hydroxyethylcellulose | 0.1 |
| Triclosan | 0.1 |
| Citric acid | 0.02 |
| Preservative | q.s |
| Hydrergy | 4 |
| Sepilift DPHP | 3.5 |
| Sodium Ascorbyl Phosphate | 1.25 |

### Method

### Stage 1

Into the water, sodium chloride and citric acid are added and dispersed. Using a propellor stirrer, hydroxyethylcellulose is added and dispersed. This phase is then heated to 70°C.

### Stage 2

The petrolatum, cetyl alcohol, dimethicone, ceteath-20, paraffinum liquidum, theobroma cacao, Sepilift DPHP and glyceryl stearate are mixed and heated to 70°C to melt the waxes.

### Stage 3

Using a homogeniser, stage 2 is added to stage 1, this is mixed until emulsified and uniform. The emulsion is then cooled to below 35°C with stirring. The remaining materials, including the Hydrergy, Sodium Ascorbyl Phosphate are then added and mixed until uniform.

### Example 7 - Eve Cream

| | %w/w |
|---|---|
| Aqua | to 100 |
| Butylene glycol | 6 |
| Paraffinum liquidum | 5 |
| Octyl methoxycinnamate | 4 |
| Dimethicone | 2 |
| Petrolutum | 2 |
| Cetearyl octanoate | 1.8 |
| Cetearyl alcohol | 1.6 |
| Glyceryl stearate | 1.5 |
| Cetyl alcohol | 1 |
| Prunus dulcis | 1 |
| Glycerin | 0.57 |
| Hydrogenated vegetable glycerides citrate | 0.5 |
| Tocopheryl acetate | 0.5 |
| Bisabolol | 0.475 |
| Panthenol | 0.45 |
| Sodium phosphate | 0.42 |
| PEG-20 stearate | 0.4 |
| Isopropyl myristate | 0.2 |
| Carbomer | 0.15 |
| PEG-12 isostearate | 0.125 |
| Allantoin | 0.1 |
| Tetrasodium EDTA | 0.1 |
| Lactic acid | 0.088 |
| Disodium phosphate | 0.083 |
| Potassium hydroxide | 0.051 |
| Hydrergy | 4 |
| Sepilift DPHP | 3 |
| Sodium Ascorbyl Phosphate | 0.75 |
| Preservative | q.s |

### Method

### Stage 1

Into the water, citric acid, EDTA, sodium phosphate, disodium phosphate and lactic acid are added and dispersed. Using a homogeniser, carbomer is added and hydrated. The aqueous phase is then heated to 70°C.

### Stage 2

The paraffinum liquidum, octyl methoxycinnamate, dimethicone, petrolatum, cetearyl octanoate, cetearyl alcohol, glyceryl stearate, cetyl alcohol, hydrogenated vegetable glycerides citrate, tocopheryl acetate, PEG-20 stearate, Sepilift DPHP, isopropyl myristate and PEG-12 isostearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, Hydrergy are then added and mixed until uniform.

### Example 8 - Eye Cream

| | %w/w |
|---|---|
| Aqua | to 100 |
| Butylene glycol | 6 |
| Paraffinum liquidum | 5 |
| Octyl methoxycinnamate | 4 |
| Dimethicone | 2 |
| Petrolatum | 2 |
| Cetearyl octanoate | 1.8 |
| Cetearyl alcohol | 1.6 |
| Glyceryl stearate | 1.5 |
| Cetyl alcohol | 1 |
| Prunus dulcis | 1 |
| Glycerin | 0.57 |
| Hydrogenated vegetable glycerides citrate | 0.5 |
| Tocopheryl acetate | 0.5 |
| Bisabolol | 0.475 |
| Panthenol | 0.45 |
| Sodium phosphate | 0.42 |
| PEG-20 stearate | 0.4 |
| Isopropyl myristate | 0.2 |
| Carbomer | 0.15 |
| PEG-12 isostearate | 0.125 |
| Allantoin | 0.1 |
| Tetrasodium EDTA | 0.1 |
| Lactic acid | 0.088 |
| Disodium phosphate | 0.083 |
| Potassium hydroxide | 0.051 |
| Sepilift DPHP | 3 |
| Sodium Ascorbyl Phosphate | 0.75 |
| CoAxel | 10 |
| Preservative | q.s |

### Method

### Stage 1

Into the water, citric acid, EDTA, sodium phosphate, disodium phosphate and lactic acid are added and dispersed. Using a homogeniser, carbomer is added and hydrated. The aqueous phase is then heated to 70°C.

### Stage 2

The paraffinum liquidum, octyl methoxycinnamate, dimethicone, petrolatum, cetearyl octanoate, cetearyl alcohol, glyceryl stearate, cetyl alcohol, hydrogenated vegetable glycerides citrate, tocopheryl acetate, PEG-20 stearate, Sepilift DPHP, isopropyl myristate and PEG-12 isostearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, CoAxel are then added and mixed until uniform.

### Example 9 - Eve Gel

| | %w/w |
|---|---|
| Aqua | to 100 |
| PVPNA copolymer | 2 |
| Propylene glycol | 2 |
| Carbomer | 1 |
| PEG-40 hydrogenated castor oil | 1 |
| Panthenol | 1 |
| Sodium hydroxide | 0.3 |
| Phenoxyethanol | 0.2 |
| Tetrasodium EDTA | 0.1 |
| Sepilift DPHP | 3 |
| Sodium Ascorbyl Phosphate | 0.75 |
| CoAxel | 7 |

### Method

### Stage 1

The EDTA, Methyldibromo glutaronitrile, and sodium hydroxide were added to the water and dissolved. PVPNA copolymer and Carbomer were added to the water and mixed using a homogeniser to ensure that the polymers were hydrated.

### Stage 2

Sepilift DPHP was solubilised in PEG-40 hydrogenated castor oil and added to the main bulk with stirring.

### Stage 3

The remaining materials, including the Sodium Ascorbyl Phosphate, CoAxel Complex were added individually with stirring until the product was homogenous.

### Example 10 - Refreshing Cream

| | %w/w |
|---|---|
| Aqua | to 100 |
| Butylene glycol | 7.5 |
| Silica | 7.2 |
| Arabinogalactan | 5.35 |
| Dimethicone | 5.35 |
| Petrolatum | 5.35 |
| Hydrated silica | 3.75 |
| Steareth-2 | 2.7 |
| Prunus dulcis | 2.7 |
| Steareth-21 | 0.9 |
| PVP/hexadecene copolymer | 0.8 |
| Carbomer | 0.32 |
| Sodium PCA | 0.2 |
| Parfum | q.s |
| Hydroxyethylcellulose | 0.16 |
| Potassium hydroxide | 0.1 |
| Propylene glycol | 0.1 |
| Sepilift DPHP | 3 |
| Sodium Ascorbyl Phosphate | 0.75 |
| CoAxel | 7 |
| Preservative | q.s |

### Method

### Stage 1

Into the water, the carbomer is added and hydrated using a homogeniser. The aqueous phase is then heated to 70°C.

### Stage 2

The silica, arabinogalactan, PVP/hexadecene copolymer, dimethicone, petrolatum, Sepilift DPHP, hydrated silica, steareth-2 and steareth-21 are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the CoAxel, Sodium Ascorbyl Phosphate are then added and until uniform.

### Example 11 - Skin Protection Lotion

| | %w/w |
|---|---|
| Aqua | to 100 |
| Dimethicone | 5 |
| Glycerins | 3 |
| Kaolin | 3 |
| Dicaprylyl maleate | 2.5 |
| Isopropyl myristate | 2.5 |
| Stearate-2 | 2 |
| Octyl methoxycinnamate | 1 |
| Steareth-21 | 1 |
| Cetyl alcohol | 0.75 |
| Butyl methoxydibenzoylmethane | 0.5 |
| Propylene glycol | 0.5 |
| Hydroxyethylcellulose | 0.4 |
| Xanthan gum | 0.24 |
| Tetrasodium EDTA | 0.1 |
| Citric acid | 0.05 |
| Sepilift DPHP | 4.0 |
| Sodium Ascorbyl Phosphate | 0.75 |
| Hydrergy | 7 |
| Preservative | q.s |

### Method

### Stage 1

Citric acid and EDTA are added to the water and dissolved. The hydroxyethylcellulose is added and hydrated using a propellor stirrer. Xanthan gum is pre-dispersed in glycerin and added to the bulk. This is stirred until uniform. The aqueous phase is then heated to 70°C.

### Stage 2

The dimethicone, dicaprylyl maleate, Sepilift DPHP, isopropyl myristate, stearate-2, octyl methoxycinnamate, steareth-21, cetyl alcohol and butyl methoxydibenzoylmethane are mixed and heated to 70 °C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate and Hydrergy are then added and mixed until uniform.

### Example 12 - Night Cream

| | %w/w |
|---|---|
| Aqua | to 100 |
| Glycerin | 5 |
| Paraffinum liquidum | 4.5 |
| Dicaprylyl maleate | 3 |
| Dimethicone | 3 |
| Petrolatum | 3 |
| Paraffin | 2.9 |
| Cetyl alcohol | 2 |
| Steareth-2 | 2 |
| Glyceryl stearate | 1.5 |
| Butyrospermum parkii | 1.5 |
| Steareth-21 | 1 |
| Cera microcristallina | 0.262 |
| Buxus chinensis | 0.5 |
| Propylene glycol | 0.48 |
| Parfum | q.s |
| Hydroxyethylcellulose | 0.3 |
| Xanthan gum | 0.25 |
| Alcohol denat. | 0.08 |
| Sodium citrate | 0.08 |
| Lecithin | 0.075 |
| BHT | 0.05 |
| Sepilift DPHP | 5.0 |
| Sodium Ascorbyl Phosphate | 0.75 |
| Hydrergy | 5 |
| Citric acid | 0.025 |
| Preservative | q.s |

### Method

### Stage 1

Citric acid and sodium citrate are added to the water and dissolved. The hydroxyethylcellulose is added and hydrated using a propellor stirrer. Xanthan gum is pre-dispersed in glycerin and added to the bulk. This is stirred until uniform. The aqueous phase is then heated to 70°C.

### Stage 2

The paraffinum liquidum, Sepilift DPHP, dicaprylyl maleate, dimethicone, petrolatum, paraffin, cetyl alcohol, steareth-2, glyceryl stearate, steareth-21, cera microcristallina and BHT are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, Hydrergy are then added and mixed until uniform.

### Example 13 - Sun Lotion for Sensitive Skin - SPF15

| | %w/w |
|---|---|
| Aqua | to 100 |
| C12-15 alkyl benzoate | 12 |
| Butylene glycol | 5 |
| Octyl methoxycinnamate | 3.8 |
| Butyl methoxydibenzoylmethane | 3 |
| Dimethicone | 2 |
| Polyglyceryl-3 methylglucose distearate | 2 |
| PVP/hexadecene copolymer | 1.75 |
| C18-36 acid glycol ester | 1.5 |
| Polysorbate 60 | 0.5 |
| Titanium dioxide | 0.3 |
| Tocopheryl acetate | 0.2 |
| Acrylates/vinyl isodecanoate crosspolymer | 0.14 |
| Potassium hydroxide | 0.035 |
| Tetrasodium EDTA | 0.02 |
| Preservative | q.s |
| Sepilift DPHP | 3.0 |
| Sodium Ascorbyl Phosphate | 0.75 |
| Hydrergy | 5 |

### Method

### Stage 1

Into the water, citric acid is added and dispersed. The acrylates/vinyl isodecanoate crosspolymer are added and dispersed using a propellor stirrer. The aqueous phase is then heated to 70°C.

### Stage 2

The C12-15 alkyl benzoate, PVP/hexadecene copolymer, octyl methoxycinnamate, Sepilift DPHP, butyl methoxydibenzoylmethane, dimethicone, polyglyceryl-3 methylglucose distearate, C18-36 acid glycol ester, polysorbate 60, titanium dioxide and tocopheryl acetate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, Hydrergy are then added and mixed until uniform.

### Example 14 - Sun Lotion for Sensitive Skin - SPF15

| | %w/w |
|---|---|
| Aqua | to 100 |
| C12-15 alkyl benzoate | 12 |
| Butylene glycol | 5 |
| Octyl methoxycinnamate | 3.8 |
| Butyl methoxydibenzoylmethane | 3 |
| Dimethicone | 2 |
| Polyglyceryl-3 methylglucose distearate | 2 |
| PVP/hexadecene copolymer | 1.75 |
| C18-36 acid glycol ester | 1.5 |
| Polysorbate 60 | 0.5 |
| Titanium dioxide | 0.3 |
| Tocopheryl acetate | 0.2 |
| Acrylates/vinyl isodecanoate crosspolymer | 0.14 |
| Potassium hydroxide | 0.035 |
| Tetrasodium EDTA | 0.02 |
| Preservative | q.s |
| Sepilift DPHP | 3.0 |
| Sodium Ascorbyl Phosphate | 0.75 |
| CoAxel | 7 |

### Method

### Stage 1

Into the water, citric acid is added and dispersed. The acrylates/vinyl isodecanoate crosspolymer are added and dispersed using a propellor stirrer. The aqueous phase is then heated to 70°C.

### Stage 2

The C12-15 alkyl benzoate, PVP/hexadecene copolymer, Sepilift DPHP, octyl methoxycinnamate, butyl methoxydibenzoylmethane, dimethicone, polyglyceryl-3 methylglucose distearate, C18-36 acid glycol ester, polysorbate 60, titanium dioxide and tocopheryl acetate are heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, CoAxel are then added and mixed. The product is then made to weight using purified water and is stirred until uniform.

### Example 15 - Sun Cream For Sensitive Skin

| | %w/w |
|---|---|
| Aqua | to 100 |
| Octyl stearate | 13.5 |
| Zinc oxide | 13.5 |
| Isopropyl myristate | 5 |
| Butylene glycol | 3 |
| lsohexadecane | 3 |
| Titanium dioxide | 2 |
| Polyglyceryl-3 oleate | 1.75 |
| Cetyl dimethicone copolyol | 1.35 |
| Magnesium sulfate | 0.75 |
| Sodium chloride | 0.75 |
| Aluminium stearate | 0.18 |
| Alumina | 0.15 |
| Lecithin | 0.13 |
| Isopropyl palmitate | 0.05 |
| Hydrergy | 5.0 |
| Sodium Ascorbyl Phosphate | 0.75 |
| CoAxel | 7 |

### Method

### Stage 1

Into the water, magnesium sulfate, sodium chloride and butylene glycol are added and dispersed. The aqueous phase is then heated to 70°C.

### Stage 2

The octyl stearate, isopropyl myristate, isohexadecane, titanium dioxide, polyglyceryl-3 oleate, cetyl dimethicone copolyol, aluminium stearate, lecithin and isopropyl palmitate are mixed and heated to 70°C to melt the waxes.

### Stage 3

Using a propellor stirrer, stage 2 is added to stage 1. Once uniform, the emulsion is transferred to a homogeniser and mixed to generate the viscosity. The emulsion is then cooled to below 35°C using stirring. The remaining materials, including the Sodium Ascorbyl Phosphate and CoAxel are then added and mixed until uniform.

### Example 16 - Anti-Ageing Foundation

| | %w/w |
|---|---|
| Aqua | to 100 |
| Butylene glycol | 9.8 |
| Cetearyl isononanoate | 4.9 |
| Dimethicone | 3.2 |
| Glycerin | 1.96 |
| Silica | 1.9 |
| Caprylic/capric triglyceride | 1.67 |
| Paraffinum liquidum | 1.67 |
| Petrolatum | 1.67 |
| Hydrogenated coco-glycerides | 1.67 |
| Cetearyl octanoate | 1.5 |
| Cetearyl alcohol | 1.35 |
| Octyl methoxycinnamate | 1.28 |
| Talc | 1 |
| Glyceryl stearate | 0.95 |
| PEG-100 stearate | 0.9 |
| Butyl methoxydibenzoylmethane | 0.6 |
| Lactic acid | 0.45 |
| Sodium polyacrylate | 0.45 |
| Boron nitride | 0.42 |
| Sodium PCA | 0.4 |
| Tocopheryl acetate | 0.4 |
| PVP/hexadecene copolymer | 0.4 |
| PEG-20 stearate | 0.33 |
| Glycolic acid | 0.2 |
| Sodium stearoyl lactylate | 0.2 |
| Isopropyl myristate | 0.17 |
| Polyaminopropyl biguanide | 0.16 |
| Tetrasodium EDTA | 0.1 |
| Xanthan gum | 0.1 |
| Citric acid | 0.06 |
| Alcohol denat. | 0.04 |
| Lecithin | 0.037 |
| Preservative | q.s |
| Sepilift DPHP | 4.0 |
| Sodium Ascorbyl Phosphate | 0.75 |
| CoAxel | 7 |

### Method

### Stage 1

Into the water, citric acid, EDTA and lactic acid are added and dispersed. Xanthan gum is pre-dispersed in butylene glycol and is added to the bulk. The aqueous phase is then heated to 70°C.

### Stage 2

The cetearyl isononanoate, dimethicone, silica, PVP/hexadecene copolymer, caprylic/capric triglyceride, paraffinum liquidum, petrolatum, hydrogenated coco-glycerides, cetearyl octanoate, Sepilift DPHP, cetearyl alcohol, octyl methoxycinnamate, talc, glyceryl stearate, PEG-100 stearate, butyl methoxydibenzoylmethane, tocopheryl acetate, sodium stearoyl lactylate, isopropyl myristate and Lecithin oil phase are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, CoAxel are then added and mixed until uniform.

### Example 17 - Anti-Ageing Foundation

| | %w/w |
|---|---|
| Aqua | to 100 |
| Butylene glycol | 9.8 |
| Cetearyl isononanoate | 4.9 |
| Dimethicone | 3.2 |
| Glycerin | 1.96 |
| Silica | 1.9 |
| Caprylic/capric triglyceride | 1.67 |
| Paraffinum liquidum | 1.67 |
| Petrolatum | 1.67 |
| Hydrogenated coco-glycerides | 1.67 |
| Cetearyl octanoate | 1.5 |
| Cetearyl alcohol | 1.35 |
| Octyl methoxycinnamate | 1.28 |
| Talc | 1 |
| Glyceryl stearate | 0.95 |
| PEG-100 stearate | 0.9 |
| Butyl methoxydibenzoylmethane | 0.6 |
| Lactic acid | 0.45 |
| Sodium polyacrylate | 0.45 |
| Boron nitride | 0.42 |
| Sodium PCA | 0.4 |
| Tocopheryl acetate | 0.4 |
| PVP/hexadecene copolymer | 0.4 |
| PEG-20 stearate | 0.33 |
| Glycolic acid | 0.2 |
| Sodium stearoyl lactylate | 0.2 |
| Isopropyl myristate | 0.17 |
| Polyaminopropyl biguanide | 0.16 |
| Tetrasodium EDTA | 0.1 |
| Xanthan gum | 0.1 |
| Citric acid | 0.06 |
| Alcohol denat. | 0.04 |
| Lecithin | 0.037 |
| Preservative | q.s |
| Sepilift DPHP | 4.0 |
| Sodium Ascorbyl Phosphate | 0.75 |
| Hydrergy | 7 |

### Method

### Stage 1

Into the water, citric acid, EDTA and Lactic acid are added and dispersed. Xanthan gum is pre-dispersed in butylene glycol and is added to the bulk. The aqueous phase is then heated to 70°C.

### Stage 2

The cetearyl isononanoate, dimethicone, Silica, PVP/hexadecene copolymer, caprylic/capric triglyceride, paraffinum liquidum, petrolatum, hydrogenated coco-glycerides, cetearyl octanoate, cetearyl alcohol, octyl methoxycinnamate, talc, glyceryl stearate, PEG-100 stearate, Sepilift DPHP, butyl methoxydibenzoylmethane, tocopheryl acetate, sodium stearoyl lactylate, isopropyl myristate and lecithin oil phase are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, Hydrergy are then added and mixed until uniform.

### Example 18 - Sun Spray - SPF15

| | %w/w |
|---|---|
| Aqua | to 100 |
| Dicaprylyl maleate | 12 |
| Butylene glycol | 5 |
| Octyl methoxycinnamate | 4 |
| Butyl methoxydibenzoylmethane | 3.5 |
| Dimethicone | 3 |
| Polyglyceryl-3 methylglucose distearate | 3 |
| Acrylates/octylacrylamide copolymer | 2 |
| C18-36 acid glycol ester | 1.5 |
| Triethanolamine | 0.5 |
| Tocopheryl acetate | 0.2 |
| Acrylates/vinyl isodecanoate crosspolymer | 0.05 |
| Tetrasodium EDTA | 0.02 |
| Potassium hydroxide | 0.015 |
| Preservative | q.s |
| Sepilift DPHP | 3.0 |
| Sodium Ascorbyl Phosphate | 0.75 |
| Hydrergy | 5 |

### Method

### Stage 1

Into the water, EDTA is added and dissolved. Acrylates/vinyl isodecanoate cross polymer are added and dispersed using a propellor stirrer. Butylene glycol is added and dispersed. The aqueous phase is then heated to 70°C.

### Stage 2

The dicaprylyl maleate, Acrylates/octylacrylamide copolymer, octyl methoxycinnamate, Sepilift DPHP, butyl methoxydibenzoylmethane, dimethicone, polyglyceryl-3 methylglucose, C18-36 acid glycol ester and tocopheryl acetate are mixed and heated to 80°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate, Hydrergy are then added and mixed until uniform.

### Example 19 - Skin pH Balancing Toner

| | %w/w |
|---|---|
| Aqua | to 100 |
| Alcohol denat. | 7.9 |
| Butylene glycol | 2 |
| Dimethicone copolyol | 1.5 |
| Sodium lactate | 0.6 |
| Glycerin | 0.5 |
| Allantoin | 0.1 |
| Propylene glycol | 0.1 |
| Lactic acid | 0.002 |
| Preservative | q.s |
| Sepilift DPHP | 5.0 |
| Sodium Ascorbyl Phosphate | 0.75 |
| CoAxel | 5 |

### Method

### Stage 1

Into the water, lactic acid and alcohol denat are separately added and dispersed until uniform. Using a propellor stirrer, all materials including the Sepilift DPHP, Sodium Ascorbyl Phosphate and CoAxel are slowly added and stirred until uniform. The product is made to weight using purified water and stirred until uniform.

### Example 20 - pH-Balanced Cleansing Lotion

| | %w/w |
|---|---|
| Aqua | to 100 |
| Paraffinum liquidum | 14 |
| Isopropyl palmitate | 7 |
| Glyceryl stearate | 2.5 |
| PEG-100 stearate | 2.5 |
| Butylene glycol | 2 |
| Hydrogenated vegetable glycerides citrate | 2 |
| Polysorbate 60 | 0.5 |
| Sorbitan stearate | 0.5 |
| Propylene glycol | 0.3 |
| Acrylates/C10-30 alkyl acrylate crosspolymer | 0.12 |
| Potassium hydroxide | 0.05 |
| Tetrasodium EDTA | 0.02 |
| Preservative | q.s |
| Sepilift DPHP | 5.0 |
| Sodium Ascorbyl Phosphate | 0.75 |
| CoAxel | 5 |

### Method

### Stage 1

Into the water, EDTA is added and dispersed. Butylene glycol is then added and dispersed. The aqueous phase is then heated to 70°C.

### Stage 2

The paraffinum liquidum, Sepilift DPHP, isopropyl palmitate, glyceryl stearate, PEG-100 stearate, hydrogenated vegetable glycerides citrate, polysorbate 60 and sorbitan stearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Sodium Ascorbyl Phosphate and CoAxel are then added and mixed until uniform.

### Example 21 Night Cream

| | %w/w |
|---|---|
| Aqua | to 100% |
| Glycerin | 7.00 |
| Butyrospermum parkii | 5.00 |
| Polyacrylamide emulsion | 4.00 |
| Cyclopentasiloxane | 3.25 |
| Steareth-21 | 2.45 |
| C12-15 alkyl benzoate | 2.00 |
| Caprylic/capric triglyceride | 2.00 |
| Cyclohexasiloxane | 1.75 |
| Dimethicone | 1.75 |
| Cetyl alcohol | 1.60 |
| Steareth-2 | 1.50 |
| Glyceryl stearate | 1.50 |
| PVP/hexadecene copolymer | 1.00 |
| Sepilift DPHP | 0.50 |
| Hydregy | 3.00 |
| Butylene glycol | 0.30 |
| Dimethiconol | 0.25 |
| Sodium ascorbyl phosphate | 0.25 |
| Acrylates/vinyl isodecanoate crosspolymer | 0.10 |
| Tetrasodium EDTA | 0.05 |
| Potassium hydroxide | 0.03 |
| Carbomer | 0.015 |
| Palmitoyl pentapeptide-3 | 0.00015 |
| Preservative | q.s |
| Fragrance (parfum) | q.s |

### Method

### Stage 1

Mix together C12-C15 alkyl benzoate, dimethicone, Butyrospermum parkii, caprylic/capric triglyceride, steareth-2, steareth-21,cetyl alcohol, glyceryl stearate, PVP/hexadecene copolymer, Sepilift and heat to 70-75°C to melt the waxes. Add acrylates/vinyl isodecanoate copolymer and homogenise for 2 minutes.

### Stage 2

Add glycerin, potassium hydroxide, tetrasodium EDTA and butylene glycol to water and heat to 70-75°C.

### Stage 3

Add Stage 1 to stage 2 and homogenise for 2 minutes. Add cyclopentasiloxane, cyclohexasiloxane, dimethicone and dimethiconol and stir. Cool to below 40°C.

### Stage 4

Add palmitoyl pentapeptide-3, Hydrergy, sodium ascorbyl phosphate, preservative and perfume and mix. Add polyacrylamide and mix until uniform.

### Example 22 Eve Cream

| | %w/w |
|---|---|
| Aqua | to 100% |
| Glycerin | 7.00 |
| Cyclopentasiloxane | 3.25 |
| Butyrospermum parkii | 2.50 |
| Cyclohexasiloxane | 1.75 |
| Stearic acid | 1.50 |
| Polyacrylamide emulsion | 2.00 |
| Cetyl alcohol | 1.00 |
| Glyceryl stearate | 0.75 |
| PEG-100 stearate | 0.75 |
| Sepilift DPHP | 0.50 |
| Hydregy | 3.00 |
| Butylene glycol | 0.30 |
| Sodium ascorbyl phosphate | 0.25 |
| Tetrasodium EDTA | 0.05 |
| Potassium hydroxide | 0.03 |
| Hesperidin methyl chalcone* | 0.025 |
| Carbomer | 0.015 |
| Steareth-20 | 0.015 |
| Dipeptide-2* | 0.0005 |
| Palmitoyl pentapeptide-3 | 0.00015 |
| Palmitoyl tetrapeptide-3* | 0.00015 |
| Preservative | q.s |
| Fragrance (parfum) | q.s |

| | |
|---|---|
| *Eyeliss (Sederma) | |

### Method

### Stage 1

Mix together Butyrospermum parkii, stearic acid, steareth-20, cetyl alcohol, glyceryl stearate, PEG-100 stearate, and Sepilift and heat to 70-75°C to melt the waxes.

### Stage 2

Add glycerin, potassium hydroxide, tetrasodium EDTA and butylene glycol to water and heat to 70-75°C.

### Stage 3

Add Stage 1 to stage 2 and homogenise for 2 minutes. Add cyclopentasiloxane, cyclohexasiloxane, dimethicone and dimethiconol and stir. Cool to below 40°C.

### Stage 4

Add palmitoyl pentapeptide-3, hesperidin methyl chalcone, dipeptide-2, palmiyoyl tetrapeptide-3, Hydrergy, sodium ascorbyl phosphate, preservative and perfume and mix. Add polyacrylamide and mix until uniform.

### Determination of energising capability

### Resazurin staining assay

The rate at which cells in culture reduce resazurin from blue (oxidised form) to fluorescent red (reduced form) may be used to visualise the rate of energy utilisation, and hence provide an assay of energising activity for a formulation under test, as follows:
Normal human (neonatal foreskin) keratinocytes were harvested by trypsinisation, washed, counted and resuspended at 100,000 per ml. 100µl of cell suspension was then plated out into the wells of a 96 well plate in Epilife keratinocyte media (Cascade Biologics M-EPI-500) and incubated for 24 hours at 37°C with 5% CO₂ for 24 hours.
The media was removed and replaced by Epilife media containing the formulation under test. The formulation under test was incubated with the cells overnight. The media was removed and the cells washed with PBS.

The cells were then stressed with a one-hour incubation with either PBS, PBS containing 1% glucose or PBS and the test formulation. Untreated, unstressed cells were used as a control. The PBS was removed and replaced with 200µl of 10µg/ml resazurin solution (made up in Epilife).

The cells were incubated at 37°C in the resazurin solution, and the fluorescence read at 530nm excitation and 590nm emission for each well at 90 minute incubation period.

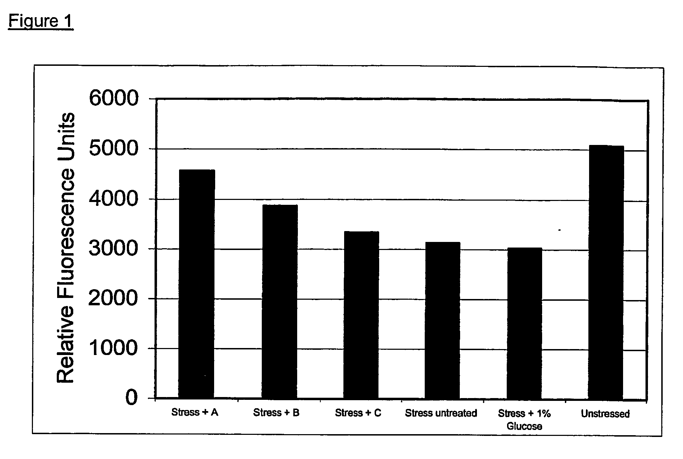

The results are shown in Figure 1, in which the test formulations are denoted as follows:
A = Sepilift, CoAxel and ascorbyl phosphate
B = Sepilift, Hydrergy and ascorbyl phosphate
C = CoAxel, Hydrergy and ascorbyl phosphate

The results show that cells treated with formulations A, B & C show increased energy compared to untreated (stress untreated) cells.

## Claims

1. A cosmetic composition comprising two or more active ingredients selected from the group consisting of
a) dipalmitoyl hydroxyproline, salts and esters thereof;
b) carnitine; and
c) an extract of *Haematococcus pluvialis.*

2. A composition as claimed in Claim 1, which comprises dipalmitoyl hydroxyproline in combination with carnitine.

3. A composition as claimed in Claim 1, which comprises dipalmitoyl hydroxyproline in combination with an extract of *Haematococcus pluvialis.*

4. A composition as claimed in any preceding claim, which further comprises one or more vitamins.

5. A composition as claimed in Claim 4, wherein said vitamins include one or more of
a) ascorbic acid (vitamin C), its salts, esters, glucosides and glucosamines, particularly sodium ascorbyl phosphate, magnesium ascorbyl phosphate and ascorbyl palmitate; and
b) tocopherol (vitamin E) and its esters, particularly tocopheryl acetate.

6. A composition as claimed in Claim 1, wherein dipalmitoyl hydroxyproline is present in an amount from 0.1 % to 10% w/w.

7. A composition as claimed in Claim 6, wherein dipalmitoyl hydroxyproline is present in an amount from 0.25% to 5% w/w.

8. A composition as claimed in Claim 1, wherein carnitine is present in the composition in an amount from 0.025% to 0.75% w/w.

9. A composition as claimed in Claim 8, wherein carnitine is present in the composition in an amount from 0.05% to 0.5% w/w.

10. A composition as claimed in Claim 1, wherein an extract of Haematococcus pluvialis is present in the composition in an amount from 0.005% to 0.1 % w/w.

11. A composition as claimed in Claim 10, wherein an extract of Haematococcus pluvialis is present in the composition in an amount from 0.01% to 0.07% w/w.

12. A composition as claimed in Claim 5, which contains vitamin C in an amount of from 0.1 to 5% w/w.

13. A composition as claimed in Claim 12, which contains vitamin C in an amount of from 0.2% to 2% w/w.

14. A composition as claimed in Claim 5, which contains vitamin E in an amount of from 0.01 % and 1 % w/w.

15. A composition as claimed in Claim 14, which contains vitamin E in an amount of from 0.05% to 0.5% w/w.

16. A composition as claimed in any preceding claim, which is selected from lipsticks, foundations, lip balms, face creams, toner cleansers, aftersuns, moisturisers, and face masks.

17. A composition as claimed in any preceding claim, which is formulated in a form selected from gels, creams, serums, pastes, lotions, milks, ointments, salves, sticks, spray, roll-on, powders, solutions, suspension dispersions and emulsions.

## Patentansprüche

1. Kosmetische Zusammensetzung, umfassend zwei oder mehr Wirkstoffe, ausgewählt aus der Gruppe, bestehend aus
a) Dipalmitoylhydroxyprolin, Salzen und Estern desselben;
b) Carnitin; und
c) einem Extrakt aus *Haematococcus pluvialis.*

2. Zusammensetzung, wie in Anspruch 1 beansprucht, welche Dipalmitoylhydroxyprolin in Kombination mit Carnitin umfasst.

3. Zusammensetzung, wie in Anspruch 1 beansprucht, welche Dipalmitoylhydroxyprolin in Kombination mit einem Extrakt aus *Haematococcus pluvialis* umfasst.

4. Zusammensetzung, wie in einem vorangehenden Anspruch beansprucht, welche weiterhin ein oder mehr Vitamine umfasst.

5. Zusammensetzung, wie in Anspruch 4 beansprucht, **dadurch gekennzeichnet, dass** die Vitamine einschließen einen oder mehr von
a) Ascorbinsäure (Vitamin C), ihre Salze, Ester, Glucoside und Glucosamine, im Besonderen Natriumascorbylphosphat, Magnesiumascorbylphosphat und Ascorbylpalmitat; und
b) Tocopherol (Vitamin E) und seine Ester, im Besonderen Tocopherylacetat.

6. Zusammensetzung, wie in Anspruch 1 beansprucht, **dadurch gekennzeichnet, dass** Dipalmitoylhydroxyprolin in einer Menge von 0,1 % bis 10 % w/w vorliegt.

7. Zusammensetzung, wie in Anspruch 6 beansprucht, **dadurch gekennzeichnet, dass** Dipalmitoylhydroxyprolin in einer Menge von 0,25 % bis 5 % w/w vorliegt.

8. Zusammensetzung, wie in Anspruch 1 beansprucht, **dadurch gekennzeichnet, dass** Carnitin in der Zusammensetzung in einer Menge von 0,025 % bis 0,75 % w/w vorliegt.

9. Zusammensetzung, wie in Anspruch 8 beansprucht, **dadurch gekennzeichnet, dass** Carnitin in der Zusammensetzung in einer Menge von 0,05 % bis 0,5 % w/w vorliegt.

10. Zusammensetzung, wie in Anspruch 1 beansprucht, **dadurch gekennzeichnet, dass** ein Extrakt von Haemotococcus pluvialis in der Zusammensetzung in einer Menge von 0,005 % bis 0,1 % w/w vorliegt.

11. Zusammensetzung, wie in Anspruch 10 beansprucht, **dadurch gekennzeichnet, dass** eine Extrakt von Haemotococcus pluvialis in der Zusammensetzung in einer Menge von 0,01 % bis 0,07 % w/w vorliegt.

12. Zusammensetzung, wie in Anspruch 5 beansprucht, welche Vitamin C in einer Menge von 0,1 % bis 5 % w/w enthält.

13. Zusammensetzung, wie in Anspruch 12 beansprucht, welche Vitamin C in einer Menge von 0,2 % bis 2 % w/w enthält.

14. Zusammensetzung, wie in Anspruch 5 beansprucht, welche Vitamin E in einer Menge von 0,01 % bis 1 % w/w enthält.

15. Zusammensetzung, wie in Anspruch 14 beansprucht, welche Vitamin E in einer Menge von 0,05 % bis 0,5 % w/w enthält.

16. Zusammensetzung, wie in einem vorangehenden Anspruch beansprucht, die ausgewählt ist aus Lippenstiften, Grundierungen, Lippenbalsamen, Gesichtscremes, Toner-Reinigungsmitteln, Aftersun-Feuchtigkeitsspendern und Gesichtsmasken.

17. Zusammensetzung, wie in einem vorangehenden Anspruch beansprucht, die zubereitet ist in einer Form, ausgewählt aus Gelen, Cremes, Sera, Pasten, Lotionen, Milch, Unguenta, Salben, Stiften, Sprays, Roll-on, Pulvern, Lösungen, Suspensionen, Dispersionen und Emulsionen.

## Revendications

1. Composition cosmétique comprenant deux ou plusieurs ingrédients actifs choisis dans le groupe consistant en
a) la dipalmitoylhydroxyproline, ses sels et ses esters ;
b) la carnitine ; et
c) un extrait de Haematococcus pluvialis.

2. Composition selon la revendication 1 qui comprend de la dipalmitoylhydroxyproline en combinaison avec de la carnitine.

3. Composition selon la revendication 1 qui comprend de la dipalmitoylhydroxyproline en combinaison avec un extrait de Haematococcus pluvialis.

4. Composition selon l'une quelconque des revendications précédentes qui comprend en outre une ou plusieurs vitamines.

5. Composition selon la revendication 4, où lesdites vitamines incluent un ou plusieurs parmi
a) l'acide ascorbique (vitamine C), ses sels, esters, glucosides et glucosamines,
en particulier le phosphate d'ascorbyle et de sodium, le phosphate d'ascorbyle et de magnésium et le palmitate d'ascorbyle ; et
b) le tocophérol (vitamine E) et ses esters, en particulier l'acétate de tocophéryle.

6. Composition selon la revendication 1, où la dipalmitoylhydroxyproline est présente en une quantité de 0,1 % à 10 % m/m.

7. Composition selon la revendication 6, où la dipalmitoylhydroxyproline est présente en une quantité de 0,25 % à 5 % m/m.

8. Composition selon la revendication 1, où la carnitine est présente dans la composition en une quantité de 0,025 % à 0,75 % m/m.

9. Composition selon la revendication 8, où la carnitine est présente dans la composition en une quantité de 0,05 % à 0,5 % m/m.

10. Composition selon la revendication 1, où un extrait de Haematococcus pluvialis est présent dans la composition en une quantité de 0,005 % à 0,1 % m/m.

11. Composition selon la revendication 10, où un extrait de Haematococcus pluvialis est présent dans la composition en une quantité de 0,01 % à 0,07 % m/m.

12. Composition selon la revendication 5 qui contient de la vitamine C en une quantité de 0,1 à 5 % m/m.

13. Composition selon la revendication 12, qui contient de la vitamine C en une quantité de 0,2 % à 2 % m/m.

14. Composition selon la revendication 5 qui contient de la vitamine E en une quantité de 0,01 % à 1 % m/m.

15. Composition selon la revendication 14 qui contient de la vitamine E en une quantité de 0,05 % à 0,5 % m/m.

16. Composition selon l'une quelconque des revendications précédentes qui est choisie parmi les crayons à lèvres, les fonds de teint, les baumes pour les lèvres, les crèmes pour le visage, les nettoyants toners, les après-soleil, les humectants et les masques pour le visage.

17. Composition selon l'une quelconque des revendications précédentes qui est formulée sous une forme choisie parmi les gels, les crèmes, les sérums, les pâtes, les lotions, les laits, les pommades, les baumes, les bâtons, les sprays, les roll-on, les poudres, les solutions, les dispersions de type suspension et les émulsions.
